(19) Europäisches Patentamt
European Patent Office
Office européen des brevets

(11) **EP 1 749 532 B1**

(12) **EUROPEAN PATENT SPECIFICATION**

(45) Date of publication and mention
of the grant of the patent:
**23.04.2014 Bulletin 2014/17**

(51) Int Cl.:
*A61K 31/7008* *(2006.01)*    *A61K 31/722* *(2006.01)*
*A61P 17/00* *(2006.01)*    *A61P 17/02* *(2006.01)*

(21) Application number: **05739362.1**

(22) Date of filing: **16.05.2005**

(86) International application number:
**PCT/JP2005/008884**

(87) International publication number:
**WO 2005/112948 (01.12.2005 Gazette 2005/48)**

(54) **DRUG FOR REMEDY OR TREATMENT OF WOUND**

ARZNEIMITTEL ZUR HEILUNG ODER BEHANLDUNG VON WUNDEN

MÉDICAMENT POUR LA GUÉRISON OU LE TRAITEMENT D'UNE BLESSURE

(84) Designated Contracting States:
**CH DE FR GB LI**

(30) Priority: **21.05.2004 JP 2004151669**

(43) Date of publication of application:
**07.02.2007 Bulletin 2007/06**

(73) Proprietor: **TOTTORI UNIVERSITY**
**Tottori-shi, Tottori 680-8550 (JP)**

(72) Inventors:
• **MINAMI, Saburo**
**4-101, Koyamacho-Minami**
**Tottori-shi, Tottori-Ken (JP)**
• **OKAMOTO, Yoshiharu**
**Tottori-shi**
**Tottori-ken (JP)**

(74) Representative: **Katzameyer, Michael et al**
**v. Bezold & Partner**
**Patentanwälte**
**Akademiestrasse 7**
**80799 München (DE)**

(56) References cited:
WO-A1-03/015797    WO-A1-03/026677
JP-A- 10 175 857    JP-A- 10 287 570
JP-A- 63 501 077    JP-A- 2001 002 551
JP-A- 2002 534 369    JP-A- 2003 183 296
JP-A- 2003 507 400    JP-A- 2004 002 369
JP-A- 2004 083 539    JP-A- 2004 107 265
JP-A- 2004 346 041    US-A- 3 232 836
US-A1- 2003 109 490

• KHOR E ET AL: "Implantable applications of chitin and chitosan" BIOMATERIALS, ELSEVIER SCIENCE PUBLISHERS BV., BARKING, GB, vol. 24, no. 13, June 2003 (2003-06), pages 2339-2349, XP004420026 ISSN: 0142-9612
• MCCARTY, M.F.: "Glucosamine for wound healing" MEDICAL HYPOTHESES, vol. 47, no. 4, October 1996 (1996-10), pages 273-275, XP002439548
• OKAMOTO Y. ET AL: 'Toshitsu Kagaku ni okeru Post Genome Kenkyu -Kinosei Kurikaeshi Tosa ni Kiso to Oyo-Chitin/Chitosan to Soho Chiyu' NIPPON NOGEI KAGAKU KAISHI vol. 78, no. 9, 01 September 2004, pages 847 - 850, XP002997083
• YANO H. ET AL: 'Effects of N-Acetyl-D-Glucosamine on Wound Healing in Rats' MIE MEDICAL JOURNAL vol. XXXV, no. 1, 1985, pages 53 - 56, XP002997084
• DATABASE CA [Online] YAKOVLEVA L.V. ET AL: 'Relation between antialternative and antiproliferative effects of indomethacin, voltaren, piroxicam and D-glucosamine', XP002997085 Database accession no. 109:16725 & FARMAKOLOGIYA I TOKSIKOLOGIYA vol. 51, no. 3, 1988, pages 70 - 72
• HOWLING G.I. ET AL: 'The effect of chitin and chitosan on the proliferation of human skin fibroblasts and keratinocytes in vitro' BIOMATERIALS vol. 22, 2001, pages 2959 - 2966, XP004301374

- **OKAMOTO Y. ET AL: 'Effects of chitin/chitosan and their oligomers/monomers on migrations of fibroblasts and vascular endothelium' BIOMATERIALS vol. 23, 2002, pages 1975 - 1979, XP004345242**
- **MORI T. ET AL: 'Effects of chitin and its derivatives on the proliferation and cytokine production of fibroblasts in vitro' BIOMATERIALS vol. 18, 1997, pages 947 - 951, XP004074547**
- **SCHMIDT R.J. ET AL: 'Biocompatibility of wound management products: the effect of various monosaccharides on L929 and 2002 fibroblast cells in culture' J.PHARM.PHARMACOL. vol. 41, 1989, pages 781 - 784, XP002997086**
- **MINAMI S. ET AL: 'Chitin-shitsu no Sosho Chiyu Sokushin Kiko' CHITIN AND CHITOSAN RESEARCH vol. 5, no. 1, 1999, pages 1 - 13, XP002997087**
- **NAKADE T. ET AL: 'Tennen Tato beta-Chitin no Inu no Sosho Chiyu Sokushin Koka' JOURNAL OF THE JAPAN VETERINARY MEDICAL ASSOCIATION vol. 49, no. 4, 1996, pages 249 - 252, XP002997088**

Remarks:
The file contains technical information submitted after the application was filed and not included in this specification

**Description**

Technical Field

The present invention relates to a agent for use in the therapy or treatment of wound. More particularly, the present invention relates to a proliferation promoting agent for epidermis keratinized cell containing N-acetyl-D-glucosamine, and a regeneration promoting agent and protecting agent for epithelium containing the proliferation promoting agent.

Background Art

Wound healing processes are classified mainly into four stages; coagulation & hemostasis period, an inflammatory period, a proliferation period and a remodeling period (epithelium re-forming period). For healing a wound region quickly with little cicatrix, quick progress of these stages and smooth transition to the subsequent stage are important. For example, delay of wound healing, pain, and cicatrisation occur by prolonging the inflammatory period or formation of excess granulation tissue in the proliferation period.

In general, chitin and chitosan are used as a therapeutic agent suitable for wound. However, a healing promotion mechanism by chitin and chitosan has not been elucidated, and thus, effective method for use of them has not been established yet.

It is known that chitin and various chitin derivatives have some influences on cells during stages of the hemostasis period, inflammatory period and proliferation period. However, regarding epithelium formation that is a final stage of the wound healing process, Howling et al. reported slightly in their studies. In Howling, G. I., Dettmar, P. W., Goddard, P. A., Hampson, F. C., Dornish, M., and Wood, E. J. 2001. The effect of chitin and chitosan on the proliferation of human skin fibroblasts and keratinocytes in vitro. Biomaterials. 22: 2959-2966, it is described that chitin does not act on basal cells forming epidermis and chitosan suppresses the proliferation ability of an epidermis keratinized cell.

Further, since chitin and chitosan are biodegraded, degradation products in various stages are also believed to relate to wound healing. However, this point is not particularly referred to.

Disclosure of Invention

An object of the present invention is to elucidate an epithelium regeneration effect of various chitin derivatives and provide an agent such as a regeneration promoting agent for epithelium which can be clinically used effectively.

According to the present invention, a use of a proliferation promoting agent for epidermis keratinized cell containing N-acetyl-D-glucosamine is provided. Further, a regeneration promoting agent for epithelium, protecting agent for epithelium and therapeutic agent for use in the treatment of a wound containing the above-mentioned proliferation promoting agent are provided.

Brief Description of Drawings

FIG. 1 is a graph showing relation of cell culture time after addition of WST-8 and absorbance.
FIG. 2 is a graph showing relation of absorbance and live cell number.
FIG. 3 is a graph showing the cell proliferation number 24 hours after addition of a chitin-based sample.
FIG. 4 is a graph showing the cell proliferation number 24 hours after addition of a chitosan-based sample.
FIG. 5 is a graph showing the cell proliferation number 48 hours after addition of a chitin-based sample.
FIG. 6 is a graph showing the cell proliferation number 48 hours after addition of a chitosan-based sample.
FIG. 7 is a graph showing transition of a left wound area of a dog.
FIG. 8 is a graph showing transition of a right femur wound area of a dog.
FIG. 9 is a photograph of a partial skin deficient mouse model.
FIG. 10A is a photograph of a mouse 8 days after application of chitin gel.
FIG. 10B is a photograph of a mouse 8 days after application of chitin gel + GlcNAc.
FIG. 10C is a photograph of a mouse 8 days after application of GlcNAc.
FIG. 10D is a photograph of a mouse 8 days after application of physiological saline.
FIG. 11 is a photograph of a mouse 16 days after application of each sample.
FIG. 12 is a photograph of skin tissue of a physiological saline-applied mouse.
FIG. 13 is a photograph of a skin tissue of a chitin gel-applied mouse.
FIG. 14 is a photograph of a skin tissue of a (chitin gel + GlcNAc)-applied mouse.

FIG. 15 is a photograph of skin a tissue of a GlcNAc-applied mouse.

Best Mode for Carrying Out the Invention

Using chitin and chitosan, and various chitin derivatives such as their oligo saccharides and monosaccharides, the present inventors have investigated a direct action thereof on a proliferation ability of an epidermis keratinized cell and an influence by its concentration. Also they have investigated an influence exerted by them on skin regeneration in animals suffering from wound on skin.

As a result, it was found that N-acetlyl-D-glucosamine which is chitin monomer increase proliferation of an epidermis keratinized cell. In addition, an effect of promoting skin regeneration on wound of animal has been found. Though application of N-acetyl-D-glucosamine as a sweetener has been reported until now, regeneration effect for an organism is not known at all. Such an action is reported for the first time by the present inventors.

In general, wound means an injury made by a cutter or the like from outside of a body, while in the present specification, it includes traumas of skin by burn and freezing.

As shown in Example 1 below, N-acetyl-D-glucosamine showed an apparent effect of proliferation of epidermis keratinized cells in an experiment in vitro.

Further, as described in Example 2, when N-acetyl-D-glucosamine was administered to a dog suffering from significant skin deficiency, it appeared that it has ideal skin regeneration effect. This dog suffered from third-degree burn. The third-degree burn is a burn to an extent requiring skin transplantation in usual cases because of complete deficiency of whole skin tissue. However, skin was regenerated quickly by treatment with N-acetyl-D-glucosamine. Thus, it was shown that N-acetyl-D-glucosamine had an effect of promoting regeneration of epithelium, particularly, skin.

Furthermore, as described in Example 3, a mouse having a wound artificially made was treated with N-acetyl-D-glucosamine, resulted in quick regeneration of skin. This wound of the mouse was made by completely excising epithelium and dermis, and further, partially excising hypodermis. Therefore, N-acetyl-D-glucosamine was shown to have an effect of promoting regeneration of tissues above hypodermis. Thus, it was shown that N-acetyl-D-glucosamine had an epithelium regeneration promoting effect, and particularly, had an effect of regenerating dermis and epidermis.

Moreover, because of these effects of N-acetyl-D-glucosamine, a protecting agent for epithelium and protecting agent for skin containing N-acetyl-D-glucosamine can also be provided.

From the above-mentioned facts, the present invention provides a proliferation promoting agent for epidermis keratinized cell containing N-acetyl-D-glucosamine. The proliferation promoting agent may further contain chitin.

According to another aspect of the present invention, there are provided a regeneration promoting agent for epithelium, regeneration promoting agent for skin and regeneration promoting agent for dermis containing the above-mentioned proliferation promoting agent.

Further, according to still another aspect of the present invention, there are provided a protecting agent for epithelium or skin containing the above-mentioned proliferation promoting agent.

According to still further another aspect of the present invention, a therapeutic agent for use in the treatment of a wound containing the above-mentioned proliferation promoting agent can be provided.

The above-mentioned regeneration promoting agents, protecting agents and therapeutic agents may contain pharmaceutically acceptable carriers, and other components. These agents may be prepared into solution, gel or cream and applied on a wound region. Alternatively, these agents may be prepared into injection and injected into a body or locally.

The above-mentioned agents of the present invention can be administered to any animals such as, for example, human, domestic animals, and pets.

Examples

[Example 1]

Human epidermis keratinized cells were cultured in the presence of chitin and chitosan as well as their derivatives, and change in proliferation ability of the cell was measured.

<Material>

As the human epidermis cell, normal human neonatal prepuce epidermis keratinized cells (NHEK(F))(Kurabo Biomedical, Osaka) were used.

A medium used for measuring NHEK(F) proliferation effect was obtained by adding 1 ml of a PSA solution (Kurabo Biomedical, Osaka) containing penicillin, streptomycin and amphoterin B, and 10% bovine fetus serum (Gibco, USA) to 500 ml of a basic medium for human epidermis keratinized cell HuMedia-KB2 (Kurabo Biomedical, Osaka).

As the medium for NHEK(F) proliferation, a proliferation medium for human epidermis keratinized cell HuMedia-KG2 (Kurabo Biomedical, Osaka) was used. HuMedia-KG2 was obtained by adding insulin (final concentration: 10 μg/ml) manufactured by Kurabo Biomedical as a microdose proliferation factor, a human recombinant type epithelium growth factor (hEGF) (final concentration: 0.1 ng/ml), hydrocortisone (0.5 μg/ml), and gentamicin (final concentration: 50 μg/ml) and amphoterin B (final concentration: 50 ng/ml) as an antimicrobacterial agent, and bovine pituitary gland extract (BPE) (final concentration: 0.4% v/v), to the basic medium HuMedia-KB2.

As other reagents for cell passage, trypsin (Kurabo Biomedical, Osaka), HEPES (Kurabo Biomedical, Osaka) and a trypsin neutralization solution (Kurabo Biomedical, Osaka) were used.

As the reagent for measuring cell proliferation, Cell Counting Kit-8 (Dojin Kagaku, Tokyo) was used.

As the chitin-based sample, the following three compounds were used.

a) Chitin

A chitin suspension aseptically prepared, Lot No. F9L01Z (stock solution: 20 mg/ml) (Sun Five K.K., Tottori) was diluted in 1000, 100, 10 and 1 μg/ml of sterile physiological saline (Otsuka Pharmaceutical Co. Ltd., Tokyo) and used. The chitin was an α-chitin and had a molecular weight of about 300000 and a degree of DAC of 8%.

b) Chitin oligomer (NACOS)

Mono to hexa-mer chitin oligo saccharide mixed powder (Yaizu Suisan Kagaku Kogyo K.K., Shizuoka) was dissolved in physiological saline so that its concentration was 10 mg/ml, and then, sterilized by filtration through a 0.22 μm millipore filter (Nippon Millipore Limited, Tokyo) to give stock solution. This stock solution was diluted to 1000, 100, 10 and 1 μg/ml with sterile physiological saline and used.

c) Chitin monomer (N-acetyl-D-glucosamine, GlcNAc)

An N-acetyl-D-glucosamine powder (Seikagaku Kogyo K.K., Tokyo) was diluted in physiological saline so that its concentration was 10 mg/ml, and then, sterilized by filtration through a 0.22 μm millipore filter (Nippon Millipore Limited, Tokyo) to give stock solution. This stock solution was diluted to 1000, 100, 10 and 1 μg/ml with sterile physiological saline and used.

As the chitosan-based sample, the following three compounds were used;

a) Chitosan

A chitosan suspension aseptically prepared, Lot No. G9L02 (stock solution: 20 mg/ml) (Sun Five K.K., Tottori) was diluted in 1000, 100, 10 and 1 μg/ml of sterile physiological saline and used. The chitosan had a molecular weight of about 80000 and a degree of DAC of 82%.

b) Chitosan oligomer (COS)

Mono to hexa-mer chitosan oligo saccharide mixed powder (Yaizu Suisan Kagaku Kogyo K.K., Shizuoka) was dissolved in physiological saline so that its concentration was 10 mg/ml, and then, sterilized by filtration through a 0.22 μm millipore filter (Nippon Millipore Limited, Tokyo) to give stock solution. This stock solution was diluted to 1000, 100, 10 and 1 μg/ml with sterile physiological saline and used.

c) Chitosan monomer (D-glucosamine, GlcN)

A D-glucosamine powder (Koyo Chemical, Tokyo) was diluted in physiological saline so that its concentration was 10 mg/ml, and then, sterilized by filtration through a 0.22 μm millipore filter (Nippon Millipore Limited, Tokyo) to give stock solution. This stock solution was diluted to 1000, 100, 10 and 1 μg/ml with sterile physiological saline and used.

As a control, sterile physiological saline (Otsuka Pharmaceutical Co. Ltd.) was used.

<Method>

(1) Culturing of normal human neonatal prepuce epidermis keratinized cell, and preparing of cell suspension

Cells were cultured under 37°C, 5% $CO_2$ and wet environments using a medium for proliferation and a flask for culturing (Lot No. 055522) (Nalge Nunc International, Denmark), and passed to quaternary culture and used in an experiment.

The medium was aspirated when proliferation of cells reach sub-confluent (60 to 80% of the flask bottom area), then, inside of the vessel was washed with HEPES, and 2 ml of trypsin was added to cause release of cells from the vessel bottom. The released cells were recovered by HEPES and added into the neutralization solution to terminate the reaction of trypsin, and then, precipitated by centrifugal separation (1100 rpm, 5 minutes), and the supernatant was discarded. The recovered cells were re-floated on a medium for proliferation, the live cell number was counted by a trypan blue discharge test, and then, transplanted into another flask for culturing so that the live cell concentration to the flask bottom area was 2500 cells/cm$^3$.

For measurement, cells were centrifugally separated, then, re-floated on a medium for measurement and adjusted at 30000 cells/ml to give a cell suspension which was then used.

(2) Cell proliferation measurement assay

Into each well of a 96-well multi-plate (Sumitomo Bakelite Co. Ltd., Tokyo), the cell suspension prepared in (1) was disseminated each in an amount of 100 $\mu$l at a concentration of 3000 cells/well. For stabilizing disseminated cells, previous culturing was carried out for 12 hours under 37°C, 5% $CO_2$ and wet environments.

Next, into each well of the multi-plate, the chitin-based samples or chitosan-based samples controlled at respective concentrations were added each in an amount of 10 $\mu$l. The final concentrations of respective samples in this operation were 100, 10, 1 and 0.1 $\mu$g/ml, respectively. True culturing was carried out for 24 hours or 48 hours under 37°C, 5% $CO_2$ and wet environments.

For counting the number of cells proliferated in the culturing, a reagent for measuring cell number (cell counting kit) WST-8 was added in an amount of 10 $\mu$l to each well of the multi-plate, and allowed to stand still under 37°C, 5% $CO_2$ and wet environments for 1 to 4 hours to cause coloration. After coloration, the absorbance was measured using EASY READER EAR340AT (Medi-Con Corporation, Osaka). Numerical treatment and statistics of the measured data were carried out using a table calculation software (Microsoft Excel xp (R)).

(3) Correlation between absorbance and cell number

[0043] For evidence of correlation between absorbance and cell number, a calibration curve was made according to the following method.

First, cell suspensions having a cell number adjusted at 30000, 15000, 7500 and 3750 cells per well were prepared, and disseminated into each well of a 96-well multi-plate and cultured for 12 hours. After culturing, cell counting kit-8 was added and absorbance was measured after 4 hours.

[Basic study in epidermis keratinized cell proliferation assay]

Determination of time necessary for coloration by reagent for measuring cell number, WST-8

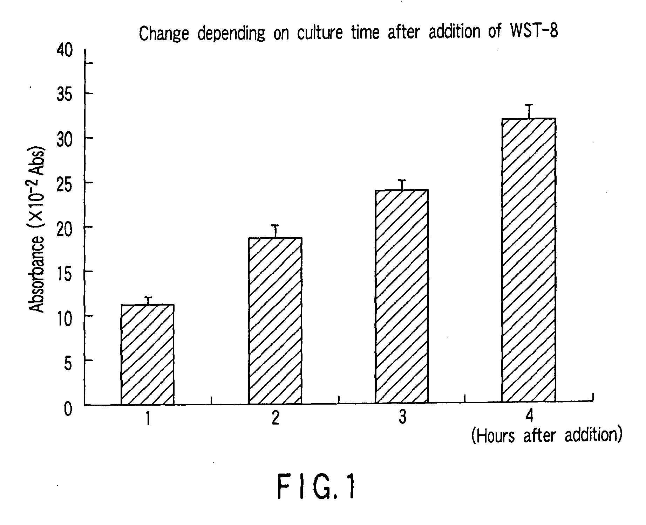

The time necessary for coloration by a reagent for measuring cell number, WST-8 is said to be 1 to 4 hours; however, its optimum time varies depending on the kind of a cell and environments. Then, change in absorbance by stand-still time after addition of the reagent for measuring cell number, WST-8 was checked using a control. The results are shown in Table 1 and FIG. 1. As apparent from FIG. 1, intensity of coloration increased and absorbance increased with the lapse of time.

Table 1 Change depending on culture time after addition of WST-8

|  | 1 hour after | 2 hour after | 3 hour after | 4 hour after |
|---|---|---|---|---|
| Average absorbance $\pm$ standard deviation ($\times$ 10$^{-2}$ Abs) | 11.45$\pm$0.56 | 18.7$\pm$1.43 | 24.05$\pm$1.01 | 31.8$\pm$1.59 |

From this result, the stand-still time after addition of a reagent for measuring cell number, WST-8, was 4 hours.

Correlation between absorbance and cell number:

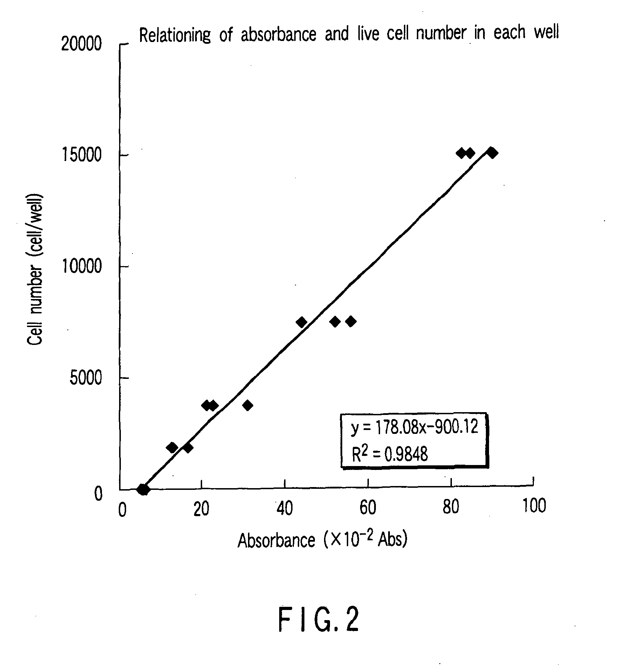

The cell number and absorbance show a proportional correlation (FIG. 2, Table 2), and this graph gave a calibration curve:

$$n = 17808a - 900.12 \ (R^2 = 0.9848)$$

{n: cell number per well, a: absorbance}

A cell number in dissemination of 3000 was subtracted from the cell number obtained from this calibration curve to give a proliferated cell number (cells/well).

Table 2 Relation of absorbance and live cell number in each well

| Cell number (cell/well) | 0 | 1875 | 3750 | 7500 | 15000 |
|---|---|---|---|---|---|
| Average absorbance ($\times$ 10) $\pm$ standard deviation ($\times$ $10^{-2}$ Abs) | 5.5±0.4 | 14.2±2.0 | 25.2±5.1 | 51.1±6.0 | 86.8±4.0 |

<Result>

Human epidermis keratinized cell proliferation assay:

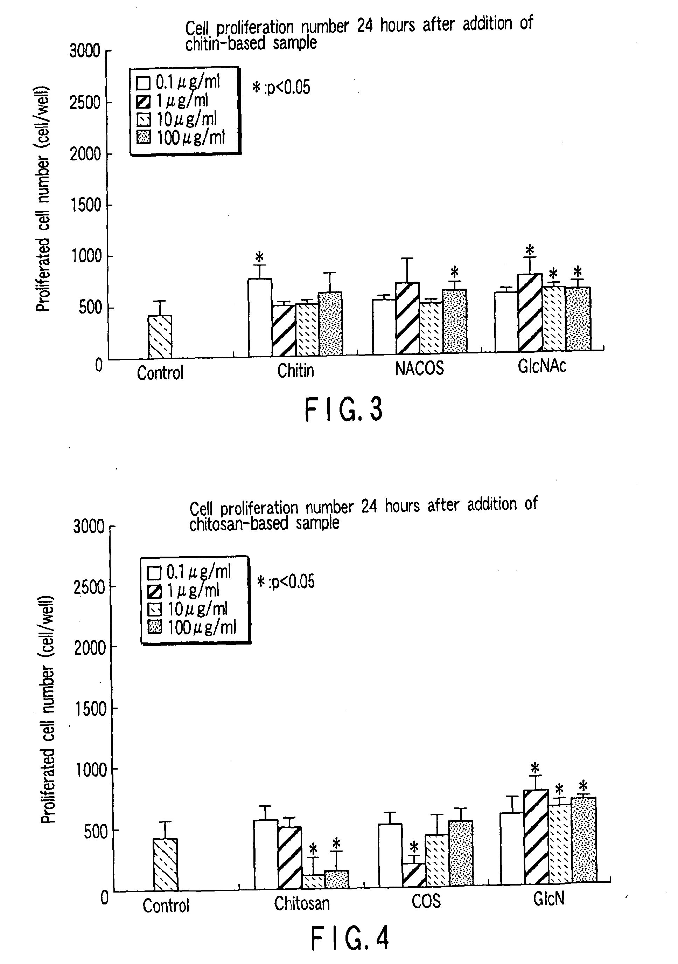

A chitin-based sample was added and culturing was carried out for 24 hours. The results are shown in Table 3 and FIG. 3. 0.1 $\mu$g/ml chitin, 100 $\mu$g/ml NACOS and 1, 10 and 100 $\mu$g/ml GlcNAc showed significant proliferation stimulating effects, respectively, as compared with the control ($p < 0.05$).

Table 3 Cell proliferation number 24 hours after addition of chitin-based sample

| | Control | Chitin | NACOS | GlcNAc |
|---|---|---|---|---|
| 0.1 $\mu$g/ml | | 769±137 | 547±42 | 601±39 |
| 1 $\mu$g /ml | 418±130 | 503±37 | 703±240 | 774±158 |
| 10 $\mu$g/ml | | 523±37 | 503±37 | 636±51 |
| 100 $\mu$g/ml | | 632±186 | 630±89 | 628±73 |
| * All of numerical values are average cell proliferation number (cell/well) $\pm$ standard deviation | | | | |

A chitosan-based sample was added and culturing was carried out for 24 hours, and the results are shown in Table 4 and FIG. 4. 10 and 100 $\mu$g/ml chitosan and COS showed significant proliferation suppressing effects, respectively, as compared with the control ($p < 0.05$). Also, 1, 10 and 100 $\mu$g/ml of GlcN showed significant proliferation stimulating effects, respectively, as compared with the control ($p < 0.05$).

Table 4 Cell proliferation number 24 hours after addition of chitosan-based sample

| | Control | Chitosan | COS | GlcN |
|---|---|---|---|---|
| 0.1 $\mu$g/ml | | 555±112 | 516±88 | 587±134 |
| 1 $\mu$g/ml | 418±130 | 495±79 | 200±62 | 766±114 |
| 10 $\mu$g/ml | | 116±140 | 423±165 | 634±65 |
| 100 $\mu$g/ml | | 142±179 | 534±95 | 689±34 |
| * All of numerical values are average cell proliferation number (cell/well) $\pm$ standard deviation | | | | |

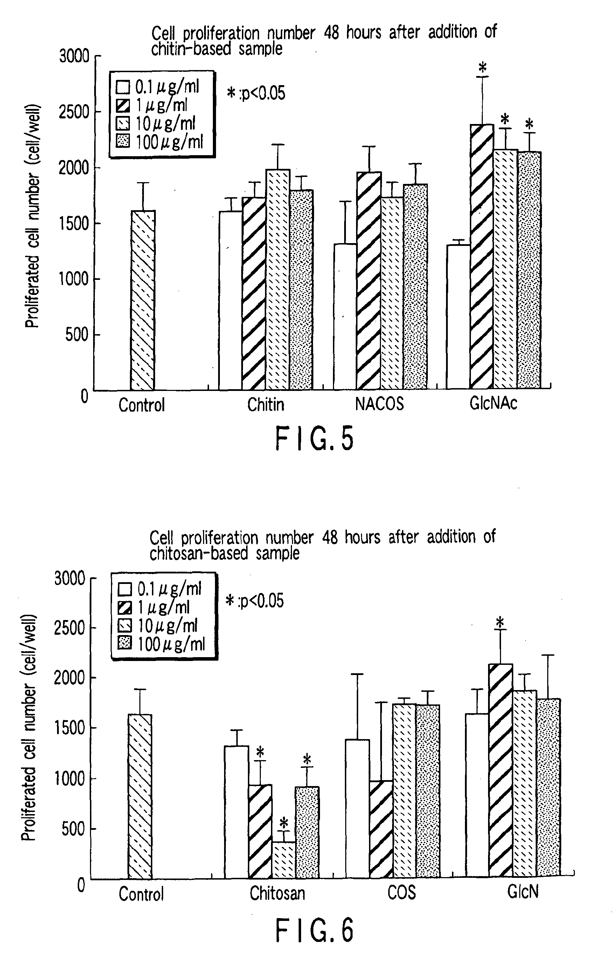

A chitin-based sample was added and culturing was carried out for 48 hours, and the results are shown in Table 5 and FIG. 5. 1, 10 and 100 $\mu$g/ml GlcNAc showed significant proliferation stimulating effects, respectively, as compared with the control ($p < 0.05$).

Table 5 Cell proliferation number 48 hours after addition of chitin-based sample

|  | Control | Chitin | NACOS | GlcNAc |
|---|---|---|---|---|
| 0.1 $\mu$g/ml | | 1616$\pm$277 | 1318$\pm$388 | 1312$\pm$169 |
| 1 $\mu$g/ml | 1629$\pm$297 | 1729$\pm$149 | 1955$\pm$241 | 2372$\pm$915 |
| 10 $\mu$g/ml | | 1977$\pm$224 | 1727$\pm$135 | 2155$\pm$175 |
| 100 $\mu$g/ml | | 1798$\pm$122 | 1852$\pm$172 | 2128$\pm$165 |
| * All of numerical values are average cell proliferation number (cell/well) $\pm$ standard deviation | | | | |

A chitosan-based sample was added and culturing was carried out for 48 hours, and the results are shown in Table 6 and FIG. 6. All concentrations (0.1, 1, 10 and 100 $\mu$g/ml) of chitosan showed significant proliferation suppressing effects as compared with the control ($p < 0.05$). On the other hand, 1 $\mu$g/ml GlcN showed a significant proliferation stimulating effect ($p < 0.05$).

Table 6 Cell proliferation number 48 hours after addition of chitosan-based sample

|  | Control | Chitosan | COS | GlcN |
|---|---|---|---|---|
| 0.1 $\mu$g/ml | | 1309$\pm$149 | 1364$\pm$659 | 1616$\pm$244 |
| 1 $\mu$g/ml | 1629$\pm$247 | 930$\pm$238 | 961$\pm$782 | 2108$\pm$344 |
| 10 $\mu$g/ml | | 363$\pm$105 | 1714$\pm$61 | 1839$\pm$160 |
| 100 $\mu$g/ml | | 908$\pm$188 | 1709$\pm$134 | 1758$\pm$424 |
| * All of numerical values are average cell proliferation number (cell/well) $\pm$ standard deviation | | | | |

From the above-mentioned experiments, a significant effect of stimulating cell proliferation ability as compared with the control is confirmed in GlcNAc and GlcN, and it is recognized that, particularly, GlcNAc shows an excellent ability to make epidermis keratinized cells proliferate, and has an effect of regenerating skin epithelium. Further, a significant cell proliferation suppressing action as compared with the control is recognized in chitosan.

Both chitin-based sample and chitosan-based sample show a tendency that a cell proliferation stimulating action becomes stronger as the molecular weight becomes smaller. There is recognized a tendency that the chitin-based sample shows a stronger cell proliferation stimulating action than the chitosan-based sample.

From the above-mentioned results, it is recognized that when chitin is decomposed more significantly, it promoted epidermis formation more, on the other hand, chitosan which is decomposed relatively moderate in an organism suppresses epidermis formation, though it shows remarkable granulation increase. This phenomenon coincides completely with a wound healing reaction to be clinically observed.

[Example 2]

GlcNAc was clinically used, and its effect was verified.

GlcNAc was sprayed together with chitin on a dog suffering from burn on the whole body by fire. The specimen was 2-old male miniature dachshund having a body weight of 3.8 kg, and suffered from third-degree burn wound at a left abdomen, third-degree burn wound at a right femur, second-degree burn wound at all palms (pads), and third-degree burn wound at 90% of a face.

In general therapy of burn, a liquid stored in a bulla is drained via a hole perforated by a clean needle and an oily ointment containing an antibiotic for preventing infection is applied, in second-degree burn. In third-degree burn, skin transplantation is essentially necessary because of skin tissue deficiency, and a strong antibiotic is applied for prevention of infection.

The drugs used in this example are a chitin powder (Sun Five K.K.), GlcNAc (Seikagaku Kogyo K.K.), chitofine (Meiji Seika kaisha, Ltd.), chitin cream (Chitosan Kowa K.K.) and antibiotic.

On 1st disease day (in first visit), a burn wound necrosis piece was removed, and a chitin powder (10 mg/cm$^2$), chitofine (100 $\mu$g/cm$^2$) and antibiotic (100 mg/cm$^2$) were sprayed on the burn region. An antibiotic ointment was applied on a rhinoscope.

On 2nd disease day, face and auricle necrosis skins were removed, and chitin, Isodine, antibiotic and GlcNAc solution (10 mg/cm$^2$) were applied.

On 3rd and later disease days, chitin, Isodine, chitin cream (1 mg/cm$^2$) (19th and later days) and GlcNAc solution were applied.

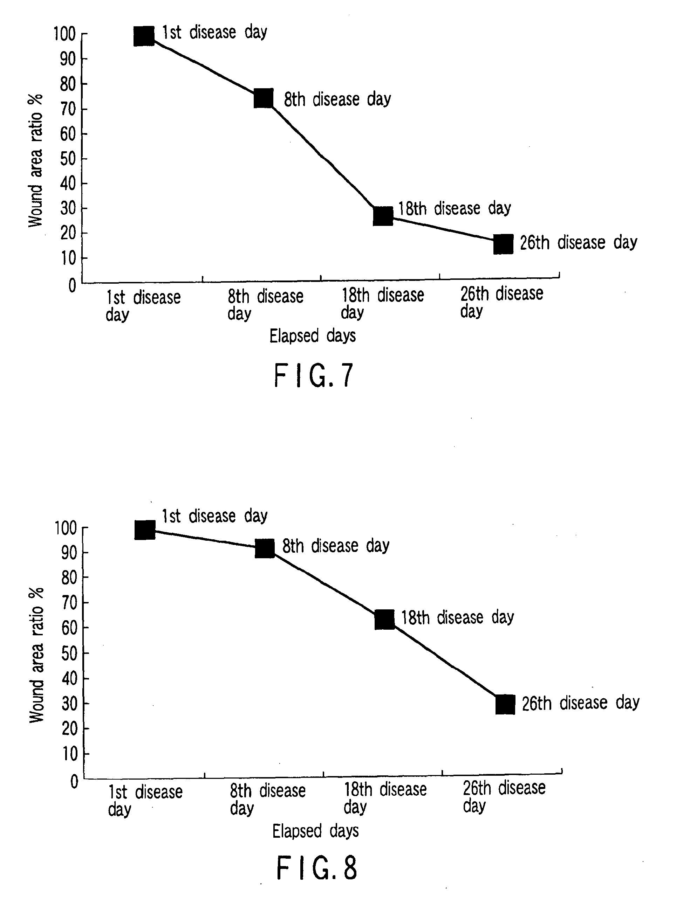

The third-degree burn wound at the left abdomen had a size of about 5.4 cm $\times$ 11.3 cm, and on 6th disease day, it was reduced to about 5 cm $\times$ 9.0 cm, and granulation was observed. On 8th disease day, the whole wound was reduced to about 4 cm $\times$ 6 cm, and granulation growth and skin epithelium neogenesis were recognized. On 18th disease day, the whole wound was further reduced to about 2.3 cm $\times$ 6 cm. On 26th disease day, the whole wound was further reduced, cicatrix was observed, and further, hair growth was recognized. The area of the above-mentioned wound at the left abdomen was 75%, 25% and 10% on 8th disease day, 18th disease day and 26th disease day, respectively, when the area on 1st disease day was 100 (FIG. 7).

The third-grade burn wound at the right femur showed necrosis of the peripheral tissue, and had a size of about 3.3 cm $\times$ 9.85 cm. On 8th disease day, wound shrinkage, granulation growth and skin epithelium neogenesis were observed. On 15th disease day, the whole wound was reduced to about 2.3 cm $\times$ 6.0 cm. On 26th disease day, the whole wound was reduced to about 0.5 cm $\times$ 9.4 cm, cicatrix formation was observed, and hair growth was recognized. The area of the above-mentioned wound at the right femur was 90%, 65% and 30% on 8th disease day, 18th disease day and 26th disease day, respectively, when the area on 1st disease day was 100 (FIG. 8).

The palms were suffered from second-degree burn at all four legs, and on 18th disease day, epidermis at a pat portion was formed, realizing healing.

The face was suffered from third-degree burn at about 90%. On 6th disease day, the whole wound was reduced, and skin epithelium neogenesis was recognized under chitin crust. On 8th disease day, risus of the face was slightly observed, but superior eyelid was formed. Natural falling of chitin was observed. On 18th disease day, granulation growth and skin epithelium neogenesis were observed, and hair growth was recognized. On 26th disease day, granulation growth and skin epithelium neogenesis were observed, and hair growth was further observed. On the head, cicatrix was partially formed.

Totally, cicatrix shrinkage was not caused, and normal skin was regenerated, and particularly, skin of the face showed complete skin regeneration and hair growth. Thus, a quick skin regeneration effect is obtained by the above-mentioned therapy combining chitin and GlcNAc. Therefore, even serious skin damage can be cured without skin transplantation by treatment with GlcNAc.

Usually, cicatrix was generated by purulence of a wound surface, excess proliferation of fibroblast, or epidermis regeneration at an insufficient granulation stage by haste wound therapy, and the like. Then, it is guessed that in from the initial period to middle period of the wound therapy process, chitosan is administered to suppress proliferation of epidermis keratinized cells and to prevent production of unnecessary keratinized substances, thereby obtaining a cicatrix preventing effect.

Therefore, it is preferable that chitin and chitosan are administered in the wound initial period, and not newly administered in the wound later period. When GlcNAc can be administered in the epithelium forming period, it can be achieved that proliferation of epidermis keratinized cell is enhanced and skin regeneration is promoted, thereby, suppressing cicatrix formation and providing quick skin regeneration therapy.

[Example 3]

Fresh skin deficient wound was made using mice and a skin regeneration promoting effect of GlcNAc was studied. As a control, chitin in the form of gel (Chitosan Kowa K.K.) and physiological saline were used.

<Experiment material and method>

Experiment material

(i) Eight 8-weeks female mice: BALB/c having a body weight of 20 to 25 g were used. In the experiment, one week-conditioning period was set.

(ii) An N-acetyl-D-glucosamine (GlcNAc) powder (Seikagaku Kogyo K.K., Tokyo) was diluted in physiological saline so that its concentration was 10 mg/ml, and then, sterilized by filtration through a 0.22 $\mu$m millipore filter to give stock solution the stock solution was then diluted with sterile physiological saline to 10 $\mu$g/ml and used.

(iii) chitin in the form of gel (Chitosan Kowa, Tokyo). Hereinafter, referred to as "chitin gel".

(iv) GlcNAc was blended in chitin gel so that its concentration was 10 $\mu$g/g and the blend was used. Hereinafter, the blend is referred to as "chitin gel + GlcNAc".

(v) Physiological saline (Otsuka Pharmaceutical Co. Ltd., Tokushima) was used as a control.

Experiment method

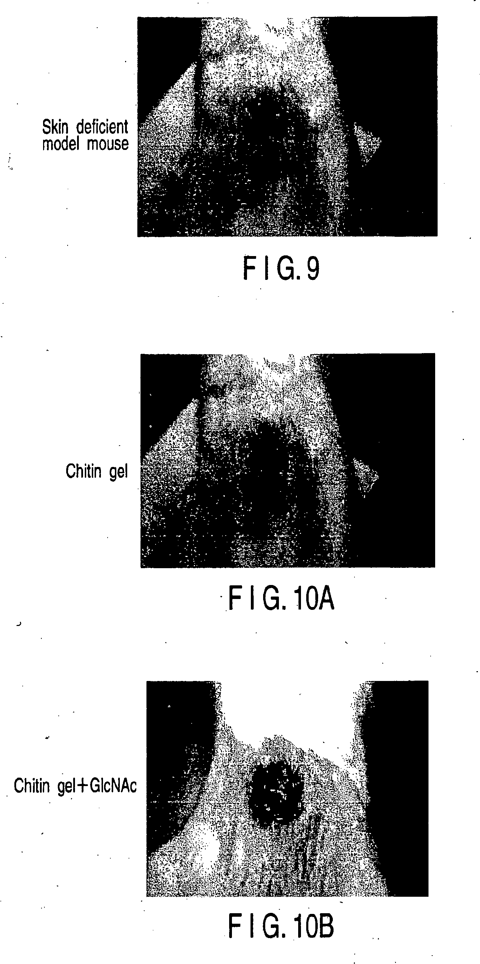

(i) Production of skin deficient model: Pentobarbital (trade name: Nembutal injection) was diluted 10-fold with physiological saline, and then, administered intraperitoneally to a mouse at 0.005 ml/g. After a sufficient anesthetic effect was confirmed, hair at the back of the mouse was shaved by a hair clipper, and the skin was disinfected with alcohol, and then, a round skin deficient region having a diameter of 5 mm was made using a 5 mm derma punch (FIG. 9). These eight mice were classified into "chitin gel group", "chitin gel + GlcNAc group", "GlcNAc group" and "physiological saline group" each containing two mice, and each group was applied once a day.

(ii) "chitin gel group" and "chitin gel + GlcNAc group" were directly applied to the skin deficient region for 7 days each at 0.1 g/head, and "GlcNAc group" and "physiological saline group" were each at 1 ml/head.

(iii) On 8th and later days, the crust formed was removed directly before application of each group, and application was carried out for further 8 days in the same manner as described above.

Visual observation

The size of the skin damage repair region was measured on 8th and 16th days using calipers.

Histological observation

Staining method: The skin deficiency repair region was collected, and fixed with a 10% neutral buffer formalin aqueous solution (formaldehyde solution). The repair region was excised to give a longitudinal section, and then, embedded in paraffin according to an ordinary method and sliced into 5 $\mu$m by a microtome. It was stained by hematoxylin-eosin staining.

<Result and discussion>

Visual observation remark

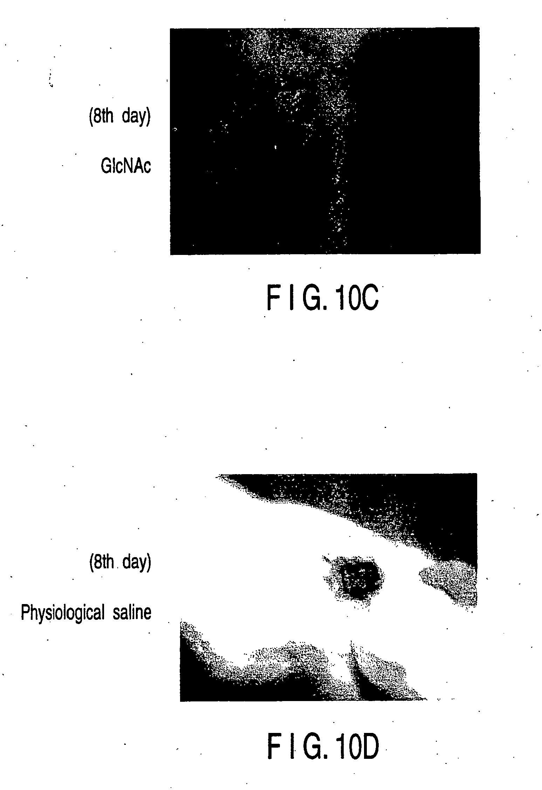

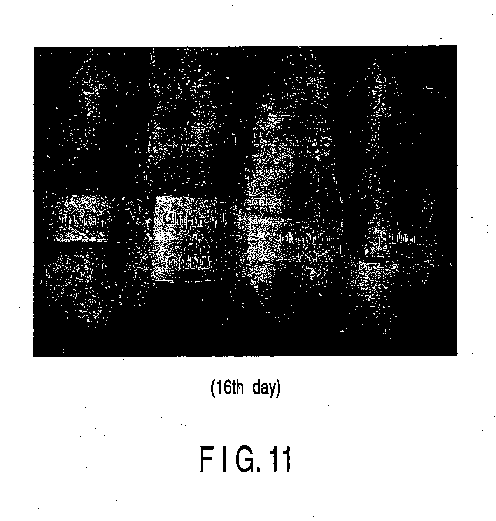

Until 7th day, a drug wad dropped from above the wound or applied. During 2nd to 3rd days, the wound was covered with a crust and hardened, as a result, reduction of the wound was not recognized in every groups. From 8th day, the crust was released every day, and administration of the drug was continued (FIGS. 10A to 10D). As a result, in "chitin gel group", epidermis formation was significantly suppressed (FIG. 11). The reason for this is believed that insoluble chitin gel apparently disturbs epidermis formation physically. In "chitin gel + GlcNAc group", on the other hand, excellent skin formation occurred and a skin regeneration effect of GlcNAc was recognized. However, excess granulation was observed in hypodermis, suggesting that granulation by chitin gel results in delay of healing in fresh wound. In "GlcNAc group", excess granulation did not occur and complete cutization was recognized, giving the most excellent healing. In "physiological saline group", epidermis formation was not completed though excess granulation was not observed.

Histological observation remark

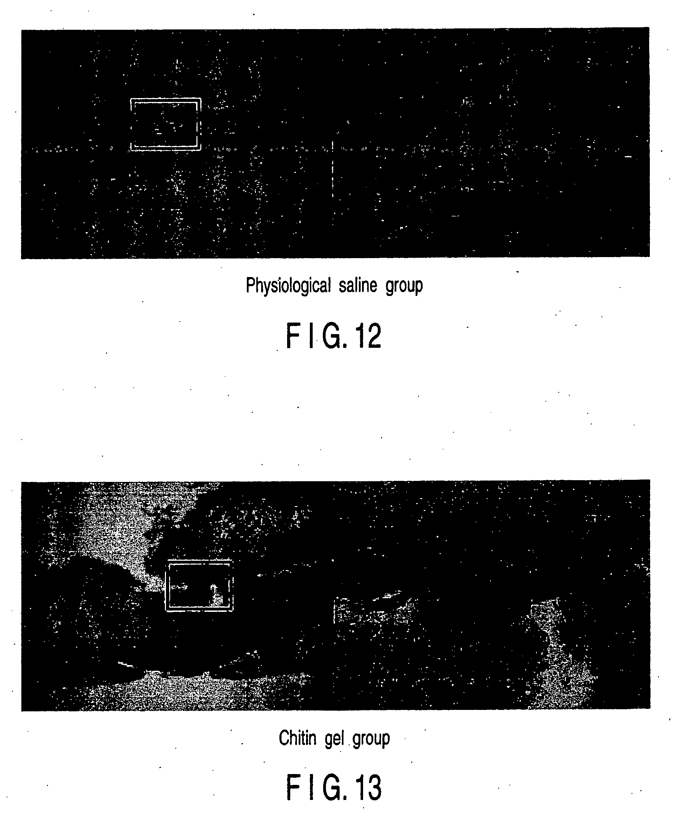

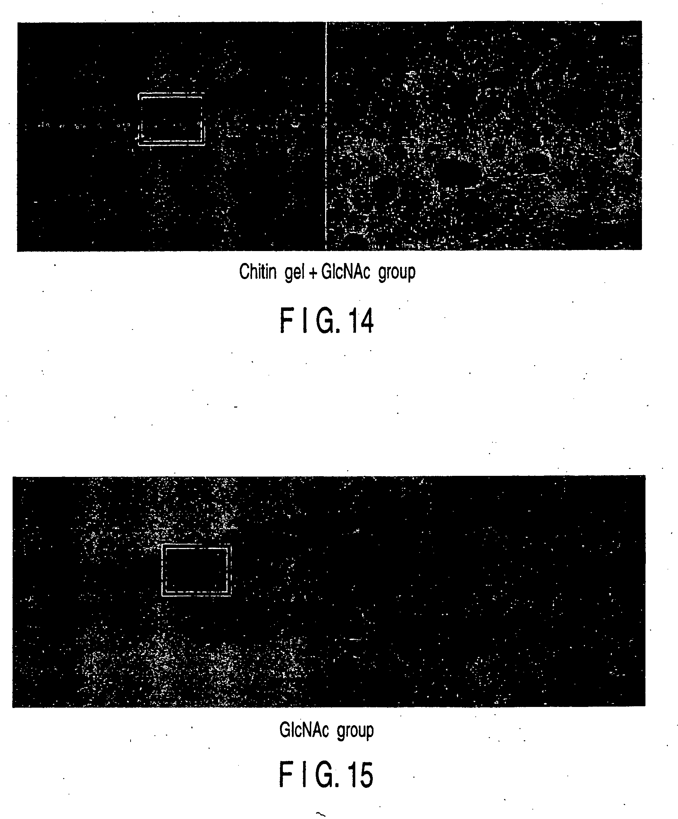

In "physiological saline group", epithelium becomes thinner toward the center of the wound, and at the center, epithelium is deficient, and continuity of skin is not completed. Though extent of inflammation is slight, degree of completion of a skin structure is low (FIG. 12). In "chitin gel group", regeneration of epidermis is disturbed by granulation, and severe inflammation continues (FIG. 13). Though regeneration of skin is completed in "chitin gel + GlcNAc group", infiltration of inflammation cells around regenerated hair follicle was recognized. The regenerated hypodermis still maintains the form of granulation, and is not completed (FIG. 14). In "GlcNAc group", skin regeneration is completed and extent of keratinization also progresses most significantly as compared with other groups. Hypodermis leaves a slight granulation tissue at the center portion, and the regenerated tissue is also completed (FIG. 15).

From the above-mentioned results, GlcNAc shows a skin regenerating effect also in a mouse, and its effect is more excellent than physiological saline and chitin gel. It was confirmed that GlcNAc shows more complete tissue regeneration also from the histological standpoint. Further, it is believed that cutization is promoted by mixing GlcNAc with chitin gel, and GlcNAc-added chitin gel is effective for therapy of deep wound.

Industrial Applicability

According to the present invention, a drug effective for therapy or treatment of wounds of animals including pets such as dogs, domestic animals, and human can be provided.

**Claims**

1. A composition in the form of a gel or cream for use in the treatment of a wound **characterized by** comprising N-acetyl-D-glucosamine (GlcNAc) and chitin and being applied to a wound site.

2. The composition for the use according to claim 1, wherein the wound is burn injuries.

3. A composition in the form of a gel or cream for use in regenerating skin **characterized by** comprising N-acetyl-D-glucosamine (GlcNAc) and chitin and being applied to a wound site.

4. The composition for the use according to claim 3, wherein the skin is epidermis.

5. The composition for the use according to claim 3, wherein the skin is epithelium

6. The composition for the use according to claim 3, wherein the skin is dermis.

**Patentansprüche**

1. Zusammensetzung in Form eines Gels oder einer Creme zur Verwendung bei der Behandlung einer Wunde, welche **dadurch gekennzeichnet ist, dass** sie N-Acetyl-D-glucosamin (GlcNAc) und Chitin umfasst und auf eine Wundstelle aufgebracht wird.

2. Zusammensetzung zur Verwendung nach Anspruch 1, wobei die Wunde eine Brandverletzung ist.

3. Zusammensetzung in Form eines Gels oder einer Creme zur Verwendung bei der Regeneration von Haut, **dadurch gekennzeichnet, dass** sie N-Acetyl-D-glucosamin (GlcNAc) und Chitin umfasst und auf eine Wundstelle aufgebracht wird.

4. Zusammensetzung zur Verwendung nach Anspruch 3, wobei die Haut Epidermis ist.

5. Zusammensetzung zur Verwendung nach Anspruch 3, wobei die Haut Epithel ist.

6. Zusammensetzung zur Verwendung nach Anspruch 3, wobei die Haut Dermis ist.

**Revendications**

1. Composition sous forme d'un gel ou d'une crème pour une utilisation dans le traitement d'une plaie, **caractérisée en ce qu'**elle comprend la N-acétyl-D-glucosamine (GlcNAc) et de la chitine et étant appliquée au site d'une plaie.

2. Composition pour une utilisation selon la revendication 1, dans laquelle la plaie représente des brûlures.

3. Composition sous forme d'un gel ou d'une crème pour une utilisation dans la régénération de la peau, **caractérisée en ce qu'**elle comprend la N-acétyl-D-glucosamine (GlcNAc) et de la chitine et étant appliquée au site d'une plaie.

4. Composition pour une utilisation selon la revendication 3, dans laquelle la peau est l'épiderme.

5. Composition pour une utilisation selon la revendication 3, dans laquelle la peau est l'épithélium.

6. Composition pour une utilisation selon la revendication 3, dans laquelle la peau est le derme.

Change depending on culture time after addition of WST-8

FIG.1

FIG. 2

FIG.3

FIG.4

EP 1 749 532 B1

Cell proliferation number 48 hours after addition of chitin-based sample

**FIG. 5**

Legend:
- □ 0.1 μg/ml
- ▨ 1 μg/ml
- ◩ 10 μg/ml
- ▦ 100 μg/ml
- *:p<0.05

Y-axis: Proliferated cell number (cell/well)

X-axis categories: Control, Chitin, NACOS, GlcNAc

Cell proliferation number 48 hours after addition of chitosan-based sample

**FIG. 6**

Legend:
- □ 0.1 μg/ml
- ▨ 1 μg/ml
- ◩ 10 μg/ml
- ▦ 100 μg/ml
- *:p<0.05

Y-axis: Proliferated cell number (cell/well)

X-axis categories: Control, Chitosan, COS, GlcN

15

F I G. 7

F I G. 8

Skin deficient
model mouse

F I G. 9

Chitin gel

F I G. 10A

Chitin gel＋GlcNAc

F I G. 10B

EP 1 749 532 B1

(8th day)

GlcNAc

F I G. 10C

(8th day)

Physiological saline

F I G. 10D

18

(16th day)

# FIG. 11

Physiological saline group

F I G. 12

Chitin gel group

F I G. 13

Chitin gel + GlcNAc group

F I G. 14

GlcNAc group

F I G. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HOWLING, G. I. ; DETTMAR, P. W. ; GODDARD, P. A. ; HAMPSON, F. C. ; DORNISH, M. ; WOOD, E. J.** The effect of chitin and chitosan on the proliferation of human skin fibroblasts and keratinocytes in vitro. *Biomaterials,* 2001, vol. 22, 2959-2966